Europäisches Patentamt

European Patent Office

Office européen des brevets

(11) Publication number: **0 050 430**
A1

# EUROPEAN PATENT APPLICATION

(21) Application number: **81304545.7**

(51) Int. Cl.³: **G 01 N 27/28**

(22) Date of filing: **01.10.81**

(30) Priority: **02.10.80 GB 8031763**

(71) Applicant: **Analox Instruments Limited, Wheatsheaf House 24 Bernard Street, Southampton S09 1QL (GB)**

(43) Date of publication of application: **28.04.82 Bulletin 82/17**

(72) Inventor: **Redstone, David, 7, Charlotte Road, Barnes, London, SW13 9QJ (GB)**

(84) Designated Contracting States: **AT BE CH DE FR GB IT LI NL SE**

(74) Representative: **Boon, Graham Anthony, Elkington and Fife High Holborn House 52/54 High Holborn, London, WC1V 6SH (GB)**

(54) **Apparatus for determining gas tension in a liquid.**

(57) An apparatus is provided for determining gas tension in a liquid. Liquid is withdrawn from a reservoir by a pump (P3) which also serves to pump a gas used for thermostatting purposes. The gas is entrained in the liquid at a junction (4), and the gas and liquid then passes through a coil (5). The thermostatting gas is then detrained at a junction (6) and pumped away by the pump (P3). The thermostatted liquid enters a chamber (16) where a reaction may take place in which the tension of gas (for example oxygen) varies. The varying gas tension in the chamber is detected by a sensor (10). One use for the apparatus is in a glucose analyzer which uses a glucose oxidation reaction.

0050430

- 1 -

<u>TITLE</u>

<u>Apparatus for determining gas tension
in a liquid</u>

This invention relates to an apparatus for determining gas tension in a liquid, especially the measurement of a change in gas tension in a stirred liquid, particularly where the change is brought about by the addition and rapid mixing with the liquid of a substance, usually another liquid, which by subsequent reaction brings about the change in gas tension. In certain circumstances the change or rate of change of the gas tension may be used to determine the concentration of one or other of the reactants and thus form the basis of an analytical system.

An example of such a reaction is that which occurs between an oxidoreductase enzyme and its substrate. A typical example is the reaction between glucose and oxygen in the presence of the enzyme glucose oxidase:

$$glucose + 0 + H_2O \xrightarrow{\text{glucose oxidase}} gluconic\ acid + H_2O_2$$

In this reaction the required oxygen is abstracted from solution which, typically, is in equilibrium with atmospheric oxygen at the outset. It is well established that if such a reaction is allowed to go to completion (end-point method), the total amount of oxygen consumed will be proportional to the total amount of glucose consumed and thus will provide a measure of the amount of glucose originally present. Furthermore, in an enzyme reaction the maximum rate of reaction is the initial rate (after a mixing transient) and it has been shown that in the reaction described the maximum rate of oxygen consumption is also proportional to the amount of glucose originally present and may be used as a measure of it.

- 2 -

The maximum rate of reaction in such a system is reached typically 10 - 20 seconds after addition of the substrate whereas in the end-point method the complete reaction may take 3 - 4 minutes. Thus the rate method has the advantage of being inherently more rapid. The rate method is described in general terms in British Patent Specification No. 1,199,565 and in an article entitled "A new and rapid method for the determination of glucose by the measurement of rate of oxygen comsumption" by Kadish et al. in "Clinical Chemistry" 14th Feb. 1968.

Analytical systems based on the principles outlined generally utilise gas-sensing electrodes to detect changes in gas tension in solution and these electrodes are generally specific for the particular gas in question. In the case of the glucose/glucose oxidase reaction a Clark-type electrode is used to monitor oxygen changes during the reaction. This electrode is membrane-covered polarographic electrode consisting of a platinum cathode and an internal silver/silver chloride reference electrode, the two being connected by a suitable electrolyte solution. The cathode is polarised at about -0.6V relative to the anode. The membrane covering the cathode is gas permeable but ion and water impermeable. The membrane is typically polytetrafluoroethylene 12 microns thick, but a variety of membrane materials and thicknesses may be used. Oxygen diffuses across the membrane to the cathode where it is reduced causing a current to flow in the system. The magnitude of this current is proportional to the amount of oxygen crossing the membrane to the cathode and this in turn is proportional to the partial pressure of oxygen outside the membrane.

An analytical apparatus utilising the principles outlined comprises a chamber to contain the reaction solution, means for filling and emptying the chamber,
an oxygen electrode or other gas sensor situated so
that it is in contact with the solution in the chamber,
means for stirring the contents of the chamber, means
for introducing a sample of one of the reactants into
the chamber and means for thermostatting the chamber,
its contents, the gas sensor and the solution to be
introduced into the chamber. It will be understood
that the liquid in the chamber may or may not be
exposed to the atmosphere depending on the construction
of the chamber. In theory any change in the oxygen
content of the liquid in the chamber might be affected
by the intrusion of oxygen from or its escape to
atmosphere. In practice it has been found that where
the chamber is relatively tall and narrow so the area
of liquid exposed to the atmosphere is minimal,
atmospheric oxygen does not interfere significantly with
the early course of the reaction when the rate of
change measurement is made. The apparatus further
comprises associated electronic apparatus, including
an amplifier to process the electrode signal including
a differentiating means and a means of determining the
maximum rate of change, means for displaying (usually
digitally) and optionally recording the values, means
for controlling pumps for filling and emptying the chamber,
means for controlling the temperature of the chamber and
other appropriate parts of the system and means for
controlling stirring in the chamber.

At the start of a typical analysis sequence by means
of the apparatus described the stirred liquid in the
chamber would be in equilibrium with atmospheric oxygen
at a specified temperature, typically 30°C. The output
of the oxygen electrode would be steady indicating that the oxygen tension in the stirred liquid was itself steady. The differentiated output of the sensor would therefore be zero or very close to zero. The liquid in the chamber would typically contain a buffer and the oxidoreductase enzyme appropriate for the substrate under test. In the example cited above this would be glucose oxidase in a suitable buffer, for example Sorensens phosphate buffer pH 6.0. An accurately metered sample of the relevant substrate, in this case glucose, is then placed in the stirred solution in the chamber and rapidly mixes with it. The reaction commences and the uptake of oxygen is monitored by the sensor. The maximum rate of uptake of oxygen occurs typically 10-15 seconds after sample injection and this maximum rate is displayed and held on a digital display. By the provision of suitable electrical adjustment the display reading can be set to correspond to the concentration value of the sample. The sample may be a solution containing an accurately known amount of glucose so setting the display to this value effectively calibrates the method. Once the maximum rate is held on the display the chamber can be emptied and refilled with fresh reagent ready for the next analysis.

Typically a complete analytical cycles takes less than 60 seconds, but before the next analysis can commence it will be necessary for the differentiated output of the oxygen sensor to be zero or very close to zero. This will only be achieved after the disturbance caused by the reaction and by the emptying and refilling of the chamber has subsided and provided that the incoming solution is in temperature and oxygen equilibrium in the chamber. If it is not, then the baseline of the rate-sensing function will be unstable and this will be reflected in increased variability and lack of accuracy in the analytical results. In practice a higher than equilibrium oxygen tension in the chamber contents will offset the baseline in such a way as to give a positive error because the oxygen tension will tend to fall and a lower than equilibrium oxygen tension will offset the baseline in such a way as to give a negative error because the oxygen tension will tend to rise. The magnitude of the error will relate directly to the offset, and the more the oxygen tension deviates from the equilibrium value the greater the error will be.

The principle reason for the deviation is that the temperature of the reagent differs from that of the chamber and electrode assembly. For example, if the temperature of the reagent is lower than the chamber it will contain more oxygen because of the greater solubility of the gas at lower temperature. When the reagent reaches the chamber it will therefore have a higher oxygen tension and this will proceed to fall because the system is open to atmosphere and in doing so will offset the baseline. In practice this difficulty is overcome either by allowing the entire system to operate at ambient temperature or by thermostatting the entire system including the reagent reservoir in, for example, an air-thermostatted box. The principle disadvantage of the former method is that ambient temperature may not be stable in a given situation. Room temperature may fall at night for instance, cooling the whole system, and when room temperature rises again the bulk of reagent may not equilibrate at the same rate as the rest of the system so that there will be delay before equilibrium is attained. The latter method of air-thermostatting may be satisfactory but suffers from the disadvantage of requiring a moving part, a fan, to distribute heat throughout the system and, because of the low thermal capacity of air, reagent placed in the box will take a long time to come to equilibrium especially if it is recently removed from refrigerator storage.

Analytical systems based on the principles outlined are often used in the context of a clinical chemistry laboratory where it may be necessary to perform an analysis of, for instance, blood glucose at short notice and at any time. It is advantageous therefore for such systems to be kept permanently on and in a high state of readiness. Given that a thermostatted system is optimal it follows that a solid-state heating system avoids the need for moving parts such as a fan, but unless the system is to be unduly bulky it poses problems concerning the thermostatting and air equilibration of a reasonably large volume of reagent. In practice the problem can be partly overcome by providing means for thermostatting a small volume of reagent which will be used immediately to replenish spent reagent in the reaction chamber. In this way by using a coil of tube which is in close contact with the reaction chamber and the gas sensor thermostatted area it is possible to bring a volume of reagent equivalent to two or three fillings of the reaction chamber into thermal equilibrium. However, in a closed system such as a tube, raising the temperature of the contents does not necessarily mean that gas tension equilibration is also achieved because egressing gas will still be trapped in the system and when introduced into the open reaction chamber will continue equilibration therein causing the baseline of the system to be unstable until the process is complete. This may take several minutes depending on the previous environment of the bulk of reagent. If it was at refrigerator temperature (0-5°C) prior to introduction into the small volume thermostatting coil then a considerable time, of the order 5 - 10 minutes, might pass before the system was sufficiently stable to use. This would apply each time the reaction chamber was filled with fresh reagent. This could be overcome by prior equilibration of the bulk of reagent but this would require a separate means for thermostatting and

- 7 -

stirring or some other method of bringing a bulk of liquid into equilibrium with air at a given temperature. In a system designed to operate at, say, 30°C it might be acceptable to allow the bulk of reagent to equilibrate at room temperature provided that this was not less than about 20°C. However, this is less than ideal and does not provide a means of using reagent taken straight from the refrigerator storage.

According to the present invention there is provided an apparatus for determining gas tension in a liquid comprising a chamber for receiving the said liquid, means for withdrawing the said liquid or a component thereof from a reservoir and entraining gas therein, equilibrating means for permitting the gas tension in the withdrawn liquid or component to reach an equilibrium value, means for detraining the said gas from the liquid or component after equilibrium has been reached, means for introducing the liquid or component after gas detrainment into the said chamber, a sensor for measuring the gas tension in the liquid in the chamber, and means for thermostatting the chamber, the sensor, the equilibrating means, the detraining means and the introducing means.

The present invention thus provides means for both thermal and gas equilibration of a relatively small volume of reagent immediately prior to its introduction into the reaction chamber, thereby minimising the time needed for the system to achieve a steady state. Cold reagent may be used immediately without prior equilibration at room temperature and only a small volume of reagent need be kept in reservoir, the bulk being refrigerated thereby improving the storage conditions and hence the useful life of what may be relatively expensive reagent.

In the accompanying drawings:

Figure 1 is a block diagram of a complete analysis apparatus; and

Figure 2 is a schematic representation of part of an analysis apparatus.

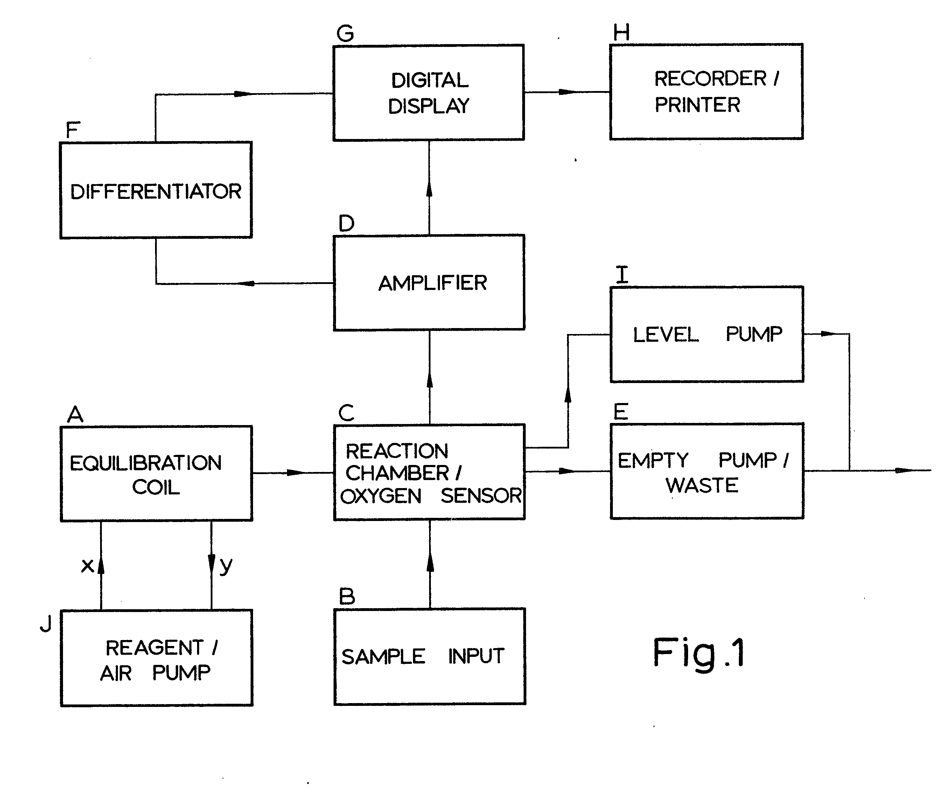

The apparatus shown in Figure 1 comprises a reaction chamber gas sensor (C), the reaction chamber being arranged to contain stirred reagent in contact with the gas sensor. A pump (E) is provided for emptying the chamber and a pump (I) is provided for levelling the liquid contents of the chamber so as to ensure that the amount of liquid in the chamber is constant each time it is refilled. A pump (J) pumps reagent into the reaction chamber via an equilibration coil (A) which forms part of the thermostatting system. Air is entrained in the reagent stream (x) so that when the reagent emerges from the coil it is in equilibrium with air at the temperature of the system. Means for detraining the air (y) from the equilibrated reagent stream is provided so that only liquid reagent enters the reaction chamber. A sample input (B) is generally in the form of manual means using a positive displacement micro-pipette. The electrical output of the sensor is processed via an amplifier (D) whence the signal may be displayed directly on the digital display (G) or after differentiation by a differentiator (F). The display reading may be recorded or printed by a recorder/printer (H) at suitable times in the analytical cycle.

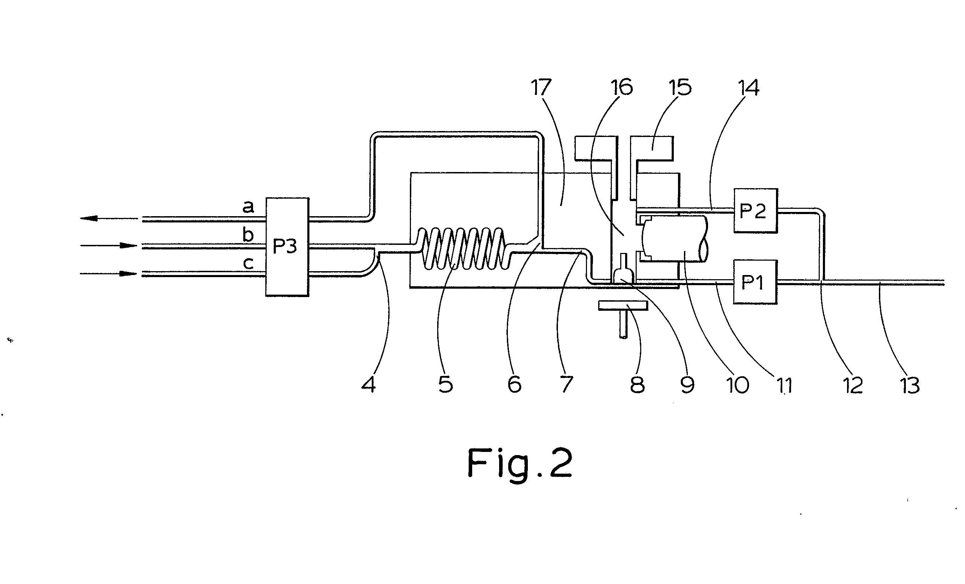

Figure 2 shows an analysis apparatus corresponding to what appears in Figure 1 but without components corresponding to (D), (F), (G), and (H). The apparatus comprises a block 17 which is a composite of high thermal conductivity materials such as aluminium, stainless steel and refilled resin. This block is thermostatted (by means not shown) to a temperature of approximately 30°C (adjustable) and it effectively thermostats the contents of the reaction chamber 16, a gas sensor, for example an oxygen sensor 10 and a coil 5 and its associated tubing and junctions. A rotating external magnet 8 causes a small encased magnetic follower 9 to rotate inside the reaction chamber 16 thus stirring the contents of the chamber. Further details of the stirring arrangement are to be found in British Patent Specification No. 1522617. The gas sensor 10 is situated in the side of the reaction chamber. A pump P1 is provided for completely draining the chamber and another pump P2 is provided for levelling the contents to ensure a consistent filling volume. Outputs from these pumps is taken to waste via a line 13. A plastic cap 15 provides a means of partially isolating the reaction chamber contents from external influences. The coil 5, which is preferably of glass, has an internal volume of approximately three to four times the volume of reagent in the reaction chamber after it has been filled and levelled. A pump P3 is provided which is a three-channel roller-type pump adapted to pump three separates fluid streams via tubes _a_, _b_ and _c_. Tube _b_ conveys reagent from a reservoir to a junction 4 where it is joined by tube _c_ which entrains air only. The volumes of reagent and air pumped to the coil via junction 4 are approximately equal and in effect the reagent stream is segmented air and reagent in approximately equal lengths. A junction 6 on the outlet of the coil has a sloping upper wall so that air tends to rise at this point where it is removed, in equivalent volume, by tube _a_ of pump P3 which is connected so that air is propelled in a direction opposite to that in tubes _b_ and _c_. After air is detrained at junction 6 reagent only is conveyed to the reaction chamber by a tube 7. Experience has shown that using this arrangement reagent entering tube _b_ at a temperature of 0 - 5°C is sufficiently equilibrated with air at 30°C by the time it reaches junction 6 that on entering the reaction chamber 16 via the tube 7 disturbance of the rate sensing baseline is minimal and the system is ready in a very short time to commence the next analysis.

In a typical analysis sequence, pump P1 operates for 8 seconds whereby the reaction chamber is completely emptied of reagent. Pumps P2 and P3 then start simultaneously and the reaction chamber is filled with equilibrated reagent to the level of tube 14 where any excess is removed by the action of pump P2. Pump P3 stops after 10 seconds operation but pump P2 continues for a further 5 seconds to ensure adequate levelling in the reaction chamber. Addition of sample via an aperture in the cap 15 initiates a reaction and when the analytical cycle is complete the emptying and refilling cycle is repeated. Any reagent entrained in tube during operation of pump P3 is returned to the reservoir.

An important advantage of the apparatus described is that complication is avoided by the use of a closed system for the air equilibration of the incoming reagent. Additional pumping means would have to be provided if an open system were used. By the use of a three channel pump for filling and air circulation only three pumps are required for the entire system. This is the minimum number that could be used in such a system even without air entrainment.

0050430

- 11 -

<u>CLAIMS:</u>

1.    An apparatus for determining gas tension in a liquid, comprising a chamber for receiving the said liquid, means for withdrawing the said liquid or a component thereof from a reservoir and entraining gas therein, equilibrating means for permitting the gas tension in the withdrawn liquid or component to reach an equilibrium value, means for detraining the said gas from the liquid or component after equilibrium has been reached, means for introducing the liquid or component after gas detrainment into the said chamber, a sensor for measuring the gas tension in the liquid in the chamber, and means for thermostatting the chamber, the sensor, the equilibrating means, the detraining means and the introducing means.

2.    An apparatus according to claim 1, wherein the means for withdrawing the said liquid or a component thereof from a reservoir and entraining gas therein comprises a liquid inlet line, a gas inlet line which has a junction with the liquid inlet line, and a single pump which acts on the liquid inlet and gas inlet lines upstream of the junction.

3.    An apparatus according to claim 2, wherein said single pump is operable to produce, downstream of the said junction, alternate segments of air and liquid.

4.    An apparatus according to claim 2 or 3, wherein a gas outlet line is arranged to carry detrained gas from the detraining means, the gas outlet line being acted on by the said single pump.

5. An apparatus according to any preceding claim, wherein the equilibrating means comprises a coil.

6. An apparatus according to any preceding claim, wherein the detraining means comprises a tube having an upwardly sloping upper wall portion.

7. An apparatus according to any preceding claim, wherein the said chamber is provided with a levelling means for producing a predetermined liquid level therein.

8. An apparatus according to claim 7, wherein the levelling means comprises a pump arranged to pump out of the chamber liquid above the predetermined level.

9. An apparatus according to claim 8, wherein the chamber is provided with a further pump for completely emptying the chamber.

10. An apparatus according to any preceding claim, wherein the said chamber is provided with means for stirring the contents thereof.

Fig.1

Fig.2

## DOCUMENTS CONSIDERED TO BE RELEVANT

| Category | Citation of document with indication, where appropriate, of relevant passages | Relevant to claim |
|---|---|---|
| | DE - A - 2 832 806 (OLYMPUS OPTICAL)<br><br>* see page 4 - page 5, paragraph 2; figure 1 *<br><br>--- | 1-4,6 |
| A | DE - A - 2 151 260 (SCHIERJOTT)<br><br>* see pages 4,5; figure 1 *<br><br>--- | 1 |
| D,A | GB - A - 1 522 617 (ANALOX INSTR.)<br><br>* see page 2, claim 1; figure 3 *<br><br>--- | 1,10 |
| P | US - A - 4 240 438 (J. STUART)<br><br>* page 1 *<br><br>--------- | 1 |

**CLASSIFICATION OF THE APPLICATION (Int. Cl. 3)**

G 01 N 27/28

**TECHNICAL FIELDS SEARCHED (Int.Cl. 3)**

G 01 N 27/28
27/56
33/48

**CATEGORY OF CITED DOCUMENTS**

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

The present search report has been drawn up for all claims

| Place of search | Date of completion of the search | Examiner |
|---|---|---|
| The Hague | 12-01-1982 | DUCHATELLIER |